# EUROPEAN PATENT SPECIFICATION

(11) **EP 2 400 914 B1**
(45) Date of publication and mention of the grant of the patent: **22.08.2018**
(21) Application number: 10706560.9
(22) Date of filing: 02.02.2010
(51) Int. Cl.: A61C 1/08, A61C 13/00

(54) **METHOD FOR PRODUCING A DENTIST TOOL**
VERFAHREN ZUR HERSTELLUNG EINES ZAHNARZTWERKZEUGS
Procédé de fabrication d'un outil de dentiste

(30) Priority: 02.02.2009 EP 09151896
(43) Date of publication of application: 04.01.2012
(60) Divisional application: 18184289.9
(73) Proprietor: VIAX DENTAL TECHNOLOGIES, LLC, Miami FL 33137 (US)
(72) Inventor: JACQUEMYNS, Evelyne, B-9830 Sint-Martens-Latem (BE)
(74) Representative: Quintelier, Claude
(86) International application number: PCT/EP2010/051261
(87) International publication number: WO 2010/086459

(56) References cited:
- WO-A2-02/34154
- DE-A1- 4 012 327
- US-A- 4 144 645
- US-A- 5 800 168
- US-A1- 2005 095 554

## Description

The present invention concerns a method for producing a dentist tool formed by an overlay to be used by a dentist in removing tooth structure from a tooth, said method being defined by claim 1. The present invention further concerns an overlay to be used by a dentist in removing tooth structure from a tooth of a patient, which tooth is to be prepared for further treatment, said dentist tool being defined by claim 13. When a patient needs a crown, bridge, onlay, inlay, veneer, or other restoring or other dental prosthesis and/or aesthetic tooth treatment, conventionally, during an initial office visit, the dentist identifies the needs of the patient and determines an appropriate treatment. During subsequent visits, in case the tooth comprises damaged and/or infected tooth structure, the dentist removes this tooth structure and reconstructs the tooth as much as possible using conventional techniques. Thereafter, the dentist performs the final preparation of the tooth for further treatment by removing tooth material, which is to be interpreted as original tooth structure and reconstructed tooth structure, with a dental cutting tool such as a high-speed drill. Relying upon eyesight and expertise, the dentist uses the drill to shape the tooth in a form that is suitable for further treatment, in particular a form onto which a crown, bridge, onlay, inlay, veneer or other tooth restoring part can be mounted. Then, the dentist takes an impression of the dental arch in which the prepared tooth is located, and sends the impression to the dental laboratory to have the dental technician make the restoration part. Using the impression of the prepared tooth, the dental technician produces a restoration part that matches the colour, geometrical and material specifications, and sends it to the dentist. At the patient's final visit, the dentist adjusts the restoration part as necessary before fitting it in place.

A further dentist tool is disclosed in EP1547544. This dentist tool decreases the impact of the experience and expertise of the dentist onto the treatment, and it is used by a dentist in boring an artificial tooth root cavity. In EP1547544, guiding means, formed by a cylindrical hole, for guiding a drill in a straight-forward way, is provided. In such a manner an artificial tooth root cavity can be bored with greater precision even by a less experienced dentist. Moreover a suitable root cavity position can be calculated based on the information processed by the computer, so as to form in the most optimal way a cavity in the jaw bone.

A drawback of the known dentist tool is that it can only be used by a dentist in boring holes into the jaw bone. This dentist tool is not suitable to be used by a dentist in preparing a tooth for further treatment.

A further dentist tool is disclosed in DE4012327, which is considered close prior art. This dentist tool is formed by a template that comprises a guiding edge for guiding a dentist cutting tool along a predetermined movement. The guiding edge is provided at a predetermined height so that besides the position, also the depth of the dentist cutting tool can be guided. DE4012327 furthermore teaches how the dentist can use the template by placing the template in the mouth of the person and, using a moving-plate fixing the tilt of the dentist cutting tool into a predetermined inclination, running with the dentist cutting tool along the guiding edge thereby removing predetermined tooth structure.

A drawback of this known dentist tool is that the tilt is, because of the moving-plate, fixed in a predetermined inclination. Thereby, the dentist tool can not guide a dentist cutting tool in a complex movement where also tilt variations occur. In particular when working with complex surfaces such as teeth, such complex movements are preferable.

It is an object of the present invention to provide method for producing a dentist tool that is suitable for guiding a dentist cutting tool along a complex movement.

To this end, the present invention is providing a method for producing a dentist tool with features as specified in independent claim 1.

The presence of the second guiding edge in the overlay results therein that the movement of a dentist cutting tool can be guided along a predetermined surface, whereas, in the prior art, the movement of the tool can only be guided along a predetermined line. Thereby also a guidance of the inclination of the dentist cutting tool can be obtained, which is not possible in the prior art. The presence of the second guiding edge results in that a dentist cutting tool can be guided along complex movements where position variations as well as tilt variations occur.

The determination of a predetermined part of tooth structure to be removed has the effect that this does not any more depend on the skills of the dentist. Determining and producing guiding edges into an overlay, which guiding edges are suitable for simultaneously contacting the dentist cutting tool, allows the cutting tool to be guided along a well determined path in an at least partially predetermined way. The overlay, having the guiding edges, accurately indicates onto the tooth the part to be removed from the tooth as determined by the computer. Moreover the tool according to the invention is provided to assist the dentist in removing tooth structure as the tool allows a dentist to remove at least part of a predetermined part of tooth structure.

It is to be noted that the term fitting is not to be interpreted in the narrow sense that the overlay should closely surround the teeth. It moreover should be interpreted as that an overlay should surround the teeth so as to be adequately in contact with the teeth and so that, once applied, it will not be released even if certain pressure, due to a normal use of the overlay, is applied.

Preferably, the method of this invention further comprises the steps of:
processing said three-dimensional data with a computer so as to determine significant dimensions and orientations for said overlay;
processing said three-dimensional data to determine said predetermined part.

Depending on the individual dental situation of a patient, the computer is programmed to determine an optimal way for shaping the tooth into a shape that is suitable for further treatment.

In a preferred embodiment of the invention, said first and said second guiding edge are designed for simultaneously contacting said dentist cutting tool at contacting points being longitudinally distanced from each other by said distance, said guiding edges thereby defining a guiding surface along which said dentist cutting tool is to be guided.

By simultaneously contacting two guiding edges with a cutting tool at contacting points being longitudinally distanced from each other, the cutting tool can be guided at least partially in a predetermined way. Preferably the cutting tool moves along the two guiding edges and thereby defines a guiding surface. This surface comprises the two guiding edges, and a cutting tool can be guided along this surface. This allows an accurate guiding in two dimensions.

Preferably, said guiding means comprise a guiding groove, which defines a path extending in a surface of said overlay and suitable for guiding said dentist cutting tool along said path in such a manner as to enable a guided removal of at least partially said predetermined part of tooth structure.

The two guiding edges, being distanced from each other, can be located one on the one side and the other on the other side of the path thereby defining the path between the two guiding edges. To this purpose, the distance between the two guiding edges should be substantially equal to a diameter of the cutting tool. Using the dentist cutting tool by simultaneously contacting the two guiding edges and following the path allows a guidance of the cutting tool at least in two dimensions. Considering a longitudinal cutting tool, the surface defined by the two guiding edges in this configuration will be substantially perpendicular to the direction of the cutting tool in use. The preferred tilt of the cutting tool, in this configuration, is perpendicular to the plane that is formed by the two guiding edges.

The two guiding edges, being distanced from each other, can also be both located on one side of the path. At least a third guiding edge will then define the other side of the path. Considering a longitudinal cutting tool in this configuration, the surface defined by the two guiding edges will be substantially parallel to the direction of the cutting tool in use. The third guiding edge in this configuration will provide a further guidance to the cutting tool thereby increasing the accuracy of the process of removal of tooth structure. Using the dentist tool by simultaneously contacting the two guiding edges and following the path in this configuration allows a guidance of the cutting tool in two dimensions and a guidance of inclination of the cutting tool.

Preferably, the method comprises the further steps of:
producing a tooth restoration part having an inner shape determined so as to fit a predetermined remaining part of tooth structure of said tooth, and having an outer shape determined so as to fit together with remaining neighbouring teeth.

An advantage of the method according to the invention is that the shape of the remaining part of tooth structure of the prepared tooth is known in advance due to the guided removal of tooth structure. This improves further treatment, such as preparing a temporary or final inlay, onlay, bridge, crown or veneer in advance based on the three-dimensional data. This results in that less office visits are required, as the dentist can both prepare the tooth and mount the tooth restoration part onto the prepared tooth in one single visit. This way of working does not only save time and thus costs, also the inconvenience for the patient is strongly decreased. Furthermore the chances that a prepared tooth gets contaminated, and the resulting complications, are reduced to a minimum.

Preferably, the method comprises the further steps of:
processing said three-dimensional data to simulate a tooth restoration part having an inner shape determined so as to fit a predetermined remaining part of tooth structure of said tooth, and having an outer shape determined so as to fit together with remaining neighbouring teeth;
producing said tooth restoration part.

Thereby, an individually designed tooth restoration part can be delivered together with the dentist tool according to the invention. This is possible because the final shape of the prepared tooth, when using the dentist tool, is known in advance. The result is that less dental visits are required to place a restoration part.

Preferably, said predetermined part is determined by:
firstly determining an outer shape of said tooth restoration part so that it fits together with remaining neighbouring teeth, and determining an inner shape of said tooth restoration part that is compatible with said outer shape;
secondly determining a shape of remaining tooth structure so that said tooth restoration part having said inner shape fits said shape of remaining tooth structure;
thirdly determining said predetermined part as being the part of tooth structure to be removed so as to have said shape of remaining tooth structure left.

Starting with determining a tooth restoration part instead of, as conventionally, preparing the tooth, has an advantage. A tooth restoration part can be determined having an outer shape so as to optimally fit together with remaining neighbouring teeth. Procedures for determination of such an outer shape are known to the person skilled in the art. An inner shape is then determined, which inner shape is compatible with said outer shape. Criteria for being compatible depend on the material that is to be used and mainly concern strength parameters, production parameters and parameters relating to the mounting of the restoration part onto the tooth and/or teeth. Preferably the inner shape is determined in such a manner that a minimum amount of material is required to produce the tooth restoration part. This at its turn results in that the remaining tooth structure, required to fit to this inner shape, is as large as possible, namely, the more material of a restored tooth is tooth restoration material, the less material of that restored tooth is tooth structure. Then the predetermined part can be determined as being the part of tooth structure to be removed so as to have said shape of remaining tooth structure left. Thus first determining the tooth restoration part, results in that a minimum of tooth structure is to be removed from the tooth and maximum conservation of the original tooth structure. As it is best to maintain as much of the original tooth structure, this is an advantage.

Providing the determined tooth restoration part having the determined outer and inner shape together with the dental tool, results in that less office visits are required because the dentist can prepare the tooth and mount the tooth restoration part onto the prepared tooth in one single visit.

Preferably said dentist tool comprises at least one further overlay, said overlay and said at least one further overlay being determined to guide a dentist tool in removing tooth structure in a plurality of stages, in which said guiding means in said overlay is provided to guide said cutting tool in one of said plurality of stages, and at least one further guiding means in said at least one further overlay is provided to guide said cutting tool in at least one further of said plurality of stages, the method further comprising the steps of:
determining at least one further overlay fitting said tooth and fitting at least a part of said neighbouring tooth;
simulating at least one further movement to be followed by said dentist cutting tool in order to remove at least one further part of said predetermined part;
determining at least one further first guiding edge within said at least one further overlay, said at least one further first guiding edge corresponding to said at least one further movement so that it is provided to contact said dentist cutting tool following said at least one further movement, thereby being provided to guide said dentist cutting tool in a predetermined position, said at least one further first guiding edge being part of said at least one further guiding means;
determining at least one further second guiding edge within said at least one further overlay that is spaced apart from said at least one further first guiding edge by a distance d of at least 0.5 mm, said at least one further second guiding edge corresponding to said at least one further movement so that it is provided to simultaneously with said at least one further first guiding edge contact said dentist cutting tool following said at least one further movement, said at least one further first and second guiding edge thereby being provided to guide said dentist cutting tool in a predetermined tilt, said at least one further second guiding edge being part of said at least one further guiding means
producing said at least one further overlay with said at least one further guiding means.

Producing a plurality of overlays instead of just one overlay provides a further flexibility in the method for producing the dentist tool. Different overlays can be provided for guiding a dentist cutting tool along different sides of the tooth to be prepared, so that for example a circumferential path can be defined by using multiple overlays each defining a different segment of the circumferential path. Multiple overlays furthermore allow to use different dental cutting tools, which is necessary in some cases. Different overlays can be provided for preparing a tooth in multiple stages, in which for example a first stage is provided to smoothen the upper part of the tooth, a second and a third stage are provided to cut away the longitudinal and transversal sides of the tooth, and a fourth and fifth stage are provided to round off the edges between the longitudinal and transversal sides each at a different side of the tooth. Preparing a tooth in stages allows each overlay to be designed having guiding edges which are particularly determined for guiding the cutting tool in a particular way characterised by the particular stage.

The invention will now be described in more details with respect to the drawings illustrating some preferred embodiments of the invention. In the drawings:
figure 1 shows a top view of an overlay with a guiding groove according to the invention;
figure 2 shows a side view of an overlay with a guiding groove placed onto a patient's teeth.
figure 3 shows a cross section of part of an overlay with guiding means according to one embodiment of the invention and a cutting tool;
figure 4 shows a cross section of part of an overlay with guiding means according to another embodiment of the invention and a cutting tool;
figure 5 shows a cross section of part of an overlay with guiding means according to the invention and a cutting tool;
figure 6 shows a cross section of part of an overlay with guiding means according to a further embodiment of the invention and a cutting tool;
figure 7 shows a set of overlays to be used by a dentist in preparing a tooth for mounting a crown or bridge.
figure 8 shows a cross-section of the overlay shown in figure 7a.

In the drawings a same reference number has been allocated to a same or analogous element.

The terms "lower", "upper" etc. and derived directional terms such as "horizontal" and "vertical" are based on a normal configuration of an overlay as shown in the drawings, wherein the overlay fits onto teeth with their root extending vertically downward. The term dentist in this text is not to be interpreted restrictive and can also be read as dental technician, dental assistant, dental auxiliary, dental therapist, dental designer etc.

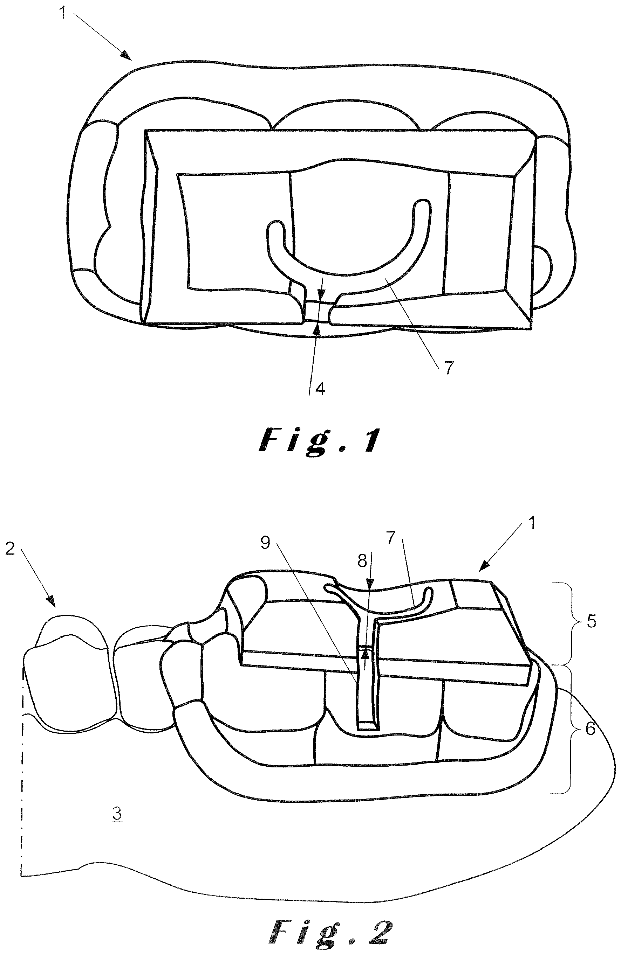

The invention provides a method for producing a dentist tool formed by an overlay 1 to be used by a dentist in removing tooth structure. Furthermore, the invention provides an overlay 1 obtainable by this method. Figure 1 and 2 show such an overlay 1 arranged onto the teeth 2 of a patient. Preferably, the invention provides a method for producing the dentist tool and a tooth restoration part in such a manner that the tooth restoration part will fit the tooth that is prepared using the dentist tool.

The dentist tool according to the invention is preferably used by a dentist in preparing a tooth for a restoration such as placing inlays and onlays, crowns, bridges, and veneers. Such restoration requires a restoration part to be produced in order to fit the tooth after the latter has been prepared. Using the dentist tool according to the invention results in that the shape of the prepared tooth, which conventionally is only known after preparation, is determined in advance. Based on this knowledge, a restoration part can be produced in advance so that this restoration part is available to the dentist even before he or she starts preparing the tooth, and can be mounted directly after preparation of the tooth. This results in that the patient suffers less inconvenience, in particular that the number of visits to the dentist are reduced. Furthermore the chances that a prepared tooth gets contaminated, and the resulting complications, are reduced to a minimum.

Preferably, preparing a tooth for further treatment is performed onto a substantially healthy tooth. When a tooth is damaged or infected, the damaged and/or infected parts of the tooth will have to be removed first, and the tooth will have to be reconstructed before preparing the tooth for further treatment. This minimizes the chance that an infection develops underneath a tooth restoration part. This also maximizes the lifetime of the tooth restoration part as the basis for the tooth restoration part, namely the tooth, does not contain damaged parts any more.

It may be possible to utilize an overlay made for one tooth in the preparation of a tooth of another patient, where such other patient's tooth has sufficiently similar dimensions and shape. Therefore, it is within the scope of this invention to have prepared some overlays which can be standardized, reused and/or reproduced. Also, it is within the scope of this invention to utilize data obtained in the preparation of prior overlays and restoration parts in the design and generation of new overlays having substantially similar dimensions and shapes.

Several methods are known for acquiring three-dimensional data from a patient's dentition. Such data can be retrieved, for example by processing photographs taken from the patient's dentition. Another method is making a physical impression of the dentition and then subsequently scanning this impression or a reversed cast of the impression. Such scanning can be conducted with a CAD/CAM 3D scanning device. Illustrative of such a device is the FreeForm® from SensAble Technologies Inc. of Woburn, Massachusetts, USA. While such a scanning device can scan a model of the dental arch with a tolerance of about 10 µm this does not take into account other error factors obtained from the making of the impression and the casting of the dental impression. In preparing the ultimate restoration part or dental prosthesis, these other error factors or tolerances must be considered..

The CAD program chooses the specific configuration for the overlay, sometimes referred to as reduction trays, and ultimately the configuration for the prepared tooth by utilizing certain protocols which are based on prior experience for preparing dental prostheses.

In one embodiment of the invention, the digital file for the subject tooth can be compared with an existing database for such prostheses. Such an illustrative database is available from Heraeus Kulzer Tooth Library of Heraeus Kulzer GmbH of Hanau Germany. This data base has also been integrated into the SensAble Dental Lab System (SDLS). A prothesis (e.g. a crown or onlay) comparable to that appropriate for the subject tooth is chosen from the Kulzer database. That prosthesis information includes the configuration and dimensions for the prepared tooth, which would correspond to the internal surface of the prosthesis. This internal configuration in the Kulzer database for that prepared crown is utilized to design the overlays (reduction trays) for use in the preparation of the patient's tooth. In the design of the overlays, fundamental principles and objectives known in the art are utilized, for example to remove the minimum amount of original tooth surface.

For example, in a set of overlays or reduction trays, there may be one overlay or tray for reduction of the medial and distel surface (front and back) and another tray for the occlusive (top) surface. Also, there could be two finishing trays which cut lingual (tongue) surface and buccal (cheek) surface of the tooth. For preparation for a crown, one would need additional trays for gingival margins.

The foregoing describes a typical use of the method but a greater or fewer number of reduction trays or overlays may be appropriate to carry out the preparation of the tooth for later installation of the dental prosthesis or restoration part.After acquiring the three-dimensional data of the tooth, this data gets processed by a computer so as to determine an overlay 1 fitting the teeth 2 of the patient. To this end, the inner shape of the overlay preferably corresponds to the outer shape of the tooth to be prepared and to the outer shape of at least part of a neighbouring tooth. Overlay should fit the teeth in such a manner that once the overlay is placed onto the teeth, it will only come off by applying a sufficiently high force to the overlay. During preparation of the tooth, small forces may be applied to the overlay, which preferably should not modify the position of the overlay on the teeth. Preferably, the overlay 1 extends over the teeth 2 so as to also cover part of the gingiva 3 or of neighboring teeth for stability purpose and protection purpose. As the dentist will use the overlay 1 for removing tooth material, it is advantageous that the overlay 1 is located onto the tooth in a stable way. As an example, an overlay 1 closely fitting the tooth, two neighbouring teeth, and part of the gingiva 3, ensures that a dentist can arrange and maintain the overlay in a stable position during removal of tooth structure. When the overlay 1 covers a part of the gingiva 3, at least this part of the gingiva 3 will be protected during the process of removing tooth structure.

It is to be noted that an overlay 1 according to the invention can cover multiple teeth at once and can be provided to be used by a dentist in preparing a plurality of teeth using one single overlay 1. This can be advantageous in the process of preparing teeth for placing veneers. Such an overlay comprises guiding means, as will be explained further, for each tooth that needs to be prepared.

The determined overlay 1 has a thickness 4 that mainly depends on the required strength thereof, and thus also the material it will be made of. An overlay 1 according to the invention can have a constant overall thickness 4. A substantially constant overall thickness is preferred for an overlay to be used by a dentist in preparing a tooth or teeth for placing veneers. An overlay to be used for preparing a tooth for placing a bridge or a crown preferably comprises an upper, guiding part 5, and a lower, supporting part 6. The supporting part 6 of the overlay 1 will ensure that the overlay 1 can be arranged in a stable way onto the teeth 2, whereas the guiding part 5 of the overlay 1 will guide the dentist cutting tool and preferably will also determine the maximal penetration dept of the cutting tool in the tooth. To this end, the thickness of the guiding part 5 of the overlay 1 will preferably be determined case by case together with the determination of the guiding means 7, as will be explained further. The thickness 4 of the supporting part 6 of the overlay 1 can be more freely chosen depending on the preferences of the dentist and/or the programmation of the computer.

After acquiring the three-dimensional data, this data gets also processed by a computer so as to determine a predetermined part of tooth structure to be removed from the tooth by the dentist. The predetermined part will be determined in view of the further treatment. If a particular shape of the prepared tooth is required for allowing further treatment, the predetermined part will be chosen so that after preparation of the tooth, this particular shape will remain.

Determining the predetermined part depends on many parameters and will differ from case to case. Parameters are the location of the tooth in the mouth of the patient, amount and location of the damaged tooth structure, distance of the tooth to neighbouring teeth, etc. In a preferred embodiment, the computer will also take into account the different parts of the tooth such as the enamel, dentin, pulp, cementum, etc. in determining the predetermined part. It is also understood within the general aspects of the invention that the patient's tooth may have to be built up through conventional techniques and materials prior to the actual step of preparation of the tooth, utilizing the overlay.

According to the invention, the computer is programmed to determine the predetermined part of tooth structure to be removed without assistance of a dentist except for what concerns the input of the initial data of the tooth. However also according to the invention, the computer can be programmed to determine the predetermined part in cooperation with a dentist, where, for example, the dentist decides on some parameters. This cooperation can be established by visualizing data relating to the tooth onto a computer display and allowing the dentist at least to enter one parameter relating to the preparation of the tooth. Preferably a preview of the tooth is visualized onto the computer display together with a preview of a simulation of the prepared tooth, so as to allow a dentist to see the impact of chosen parameters onto the prepared tooth. The latter feature provides a larger degree of freedom to the dentist and allows a less experienced dentist to consult a more experienced dentist about a case, based on the data in the computer. In this manner, the method for producing a dentist tool according to the invention allows a less experienced dentist to decide on a further treatment and prepare a tooth for further treatment with a same quality as if he or she was an experienced dentist.

In another embodiment, the computer is programmed to determine the predetermined part of tooth structure to be removed in several phases. At the end of each phase, the computer proposes a simulated solution to the dentist and requests for approval or correction of the proposed solution.

Preferably the predetermined part will be determined in several steps. In a first step, the outer shape of the tooth restoring part is to be determined so that the tooth restoring part fits with the remaining teeth and fits into the dental arch of the patient. A bite lift or other corrective amendment can be directly integrated into the design of the outer shape. Then, based on this determined outer shape, an inner shape gets determined that is compatible with the outer shape. In such a manner, a tooth restoration part can be determined having an optimal outer shape, and an optimal construction in the sense that it is strong enough and a minimum of material is needed. In a following step, the shape of remaining tooth structure is to be determined so that the inner shape of the tooth restoring part fits the remaining tooth structure after removal of tooth structure. In a final step, the predetermined part of tooth structure is determined as the part of tooth structure to be removed to have the shape of remaining tooth structure left. This way of working allows determining the outer shape of the tooth restoring part using best fit programs. Conventionally, the dentist will not follow the steps in the succession as mentioned above. The dentist will work the other way around, as the dentist will need to know the shape of the prepared tooth to determine the inner shape of the restoration part, after which the dentist will determine an appropriate outer shape. Firstly determining the tooth restoration part, according to a preferred embodiment of the invention, results in that a minimum of tooth structure is to be removed from the tooth and a maximum conservation of the original tooth structure can be obtained.

Once the predetermined part has been determined, guiding means can be determined. The purpose of the guiding means is to guide a dentist cutting tool, i.e. a drill, along a simulated movement whereby the cutting tool removes at least a part of the predetermined part. To this end, it will be understood that in some cases, the dimensions of the dentist cutting tool, such as the length, diameter, cross-sectional form, etc. will be determining for which movement is to be made with the cutting tool to remove a certain part of tooth structure. It will also be understood that in many cases, it will not be sufficient to make one single movement for removing all the predetermined part. Via computer calculation and/or simulation, one or more movements can be simulated for removing the predetermined part of tooth structure. It is to be understood that a movement in this context does not only relates to a two-dimensional position, but relates to the movement of the cutting tool in all its aspects such as horizontal movement, vertical movement and tilt movement.

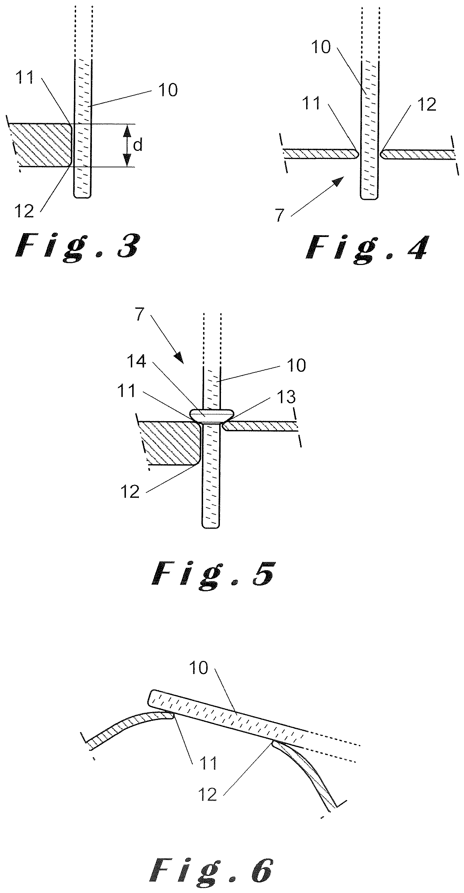

The guiding means according to the invention comprise at least two guiding edges 11, 12 (figure 3-6). Two of these guiding edges 11, 12 are spaced apart from each other with a distance d of at least 0,5 mm. The guiding edges 11, 12 are provided to simultaneously contact the cutting tool 10 thereby guiding the cutting tool 10 at least partially into a predetermined movement. It is to be understood in this context that a surface having a length and having a width which are at least 0,5 mm can be considered as the said at least two guiding edges 11, 12. Namely, the two outer edges of the surface are provided to simultaneously contact the cutting tool 10, and are spaced apart with a distance d of at least 0,5 mm.

The guiding edges 11, 12 are determined in correspondence with the simulated movement of the dentist cutting tool such that both guiding edges 11 and 12 are simultaneously in contact with the cutting tool following the movement. Preferably, the guiding edges are simultaneously and constantly in contact with the cutting tool, preferably during the whole of the movement. The contact between a guiding edge and the cutting tool, along the movement, is preferably a point of contact.

Preferably the two guiding edges 11, 12 are spaced apart from each other with a distance d of at least 1 mm, more preferably of at least 2 mm and most preferably of at least 3 mm. Increasing the distance d between the two edges 11, 12 will increase the guiding quality as it will be much easier for a dentist to control the cutting tool 10 onto larger guiding means.

The guiding edges 11, 12 are provided in such a manner as to be simultaneously contacted by the cutting tool at two contacting points. As illustrated in figure 3, preferably these contacting points considered on the cutting tool 10, are longitudinally spaced apart by the distance d. When the cutting tool 10 is arranged substantially perpendicular to the guiding edges, the distance between the contacting points will be substantially the same as the distance d between the guiding edges. Figure 3 illustrates a cutting tool 10 lying simultaneously against the two guiding edges 11, 12. The guiding edges 11, 12 in this configuration define a surface along which the cutting tool 10 can be guided.

However the guiding edges 11, 12 can also simultaneously contact the cutting tool 10 at two contacting points being located each at one side of the cutting tool 10, as illustrated in figure 4. In this configuration, the guiding edges 11, 12 define a guiding groove in which the cutting tool 10 can be guided. The guiding groove 7 is preferably determined so as to define a path laying in the upper surface of the overlay 1. The groove 7 is provided for guiding a dentist cutting tool along this path, and to this end, the groove 7 extends through the overlay so as to form a communication between the inside of the overlay and the outer environment. When more than one path is to be followed for removing the predetermined part, multiple guiding grooves 7 will have to be determined.

A combination of these two mentioned configurations is illustrated in figure 5. In this figure, a first and second guiding edge 11, 12, being the two guiding edges that are spaced apart, can be seen on the left hand side, which edges define a guiding surface. A third guiding edge 13 can be seen on the right hand side, which edge defines, together with the first and second guiding edge 11, 12, a path. This configuration therefore allows a cutting tool to be guided into both a horizontal movement and a tilt.

As can be seen in figure 5, the cutting tool 10 preferably comprises a collar 14 mounted onto the cutting tool 10. Preferably at least one of the guiding edges 11, 12, 13 is provided to guide the collar 14 of the cutting tool 10 thereby guiding the cutting tool 10 into a predetermined vertical position or height or depth. As can be seen in the figure 5, the guiding groove 7 defining a horizontal path, furthermore defines a predetermined height 8 for each horizontal location of the cutting tool, so as to guide the cutting tool 10 along a horizontal path and in a predetermined tilt, and also guiding the cutting tool in the vertical way.

Figure 6 illustrates a variant on the embodiment shown in figure 3, and shows the guiding edges 11, 12, which are provided to simultaneously contact the cutting tool at two contacting points being longitudinally distanced from each other. In this embodiment, tooth structure can be removed that is located in between the two guiding edges 11, 12. In particular in preparing a tooth for placing veneers, this embodiment will be used.

For mounting a crown or a bridge, the tooth is to be shaped into a truncated pyramid having rounded edges. To this end the outer and upper part of the tooth are to be removed. This removal of tooth structure can be guided by the dentist tool according to the invention.

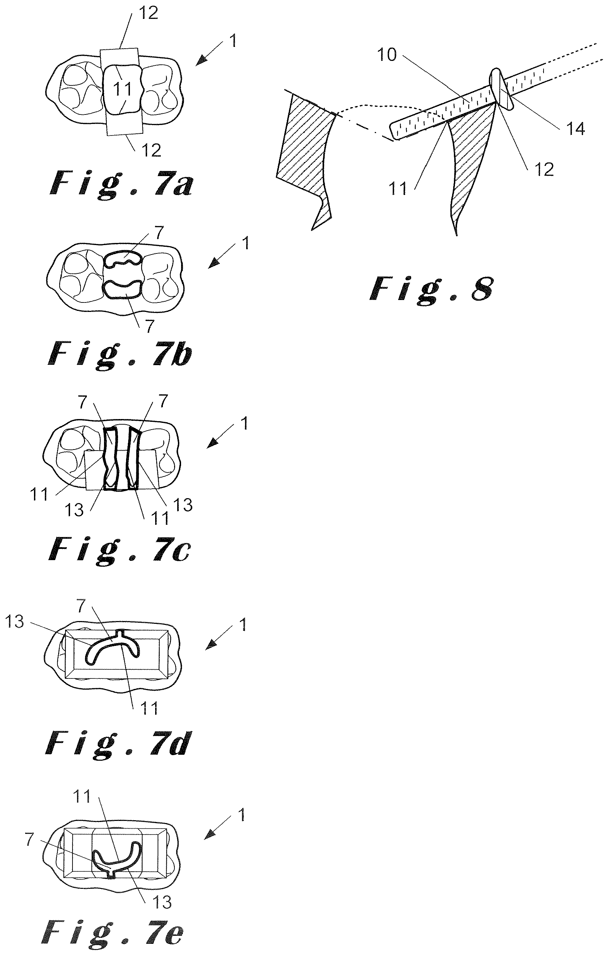

Preferably the removal of tooth structure for preparing a tooth for mounting a crown or a bridge is performed in several stages shown in figure 7. In a first stage, shown in figure 7a and figure 8, the upper part of the tooth gets smoothened. This can be done using an overlay having guiding edges 11, 12 as illustrated in figure 6. However in practice, it is preferred that the upper part gets shaped into a V-form. To this end, the upper part preferably gets smoothened using two guiding means as illustrated in figure 3, which guiding means are located on both sides of the tooth in such a manner that said guiding means are provided guide said cutting tool to cut said upper part into a V-shape.

In a second and a third stage, shown in figures 7b and 7c, the longitudinal and transversal sides of the tooth get cut, so as to obtain a truncated pyramid form. The cutting of these sides can be guided by overlays having guiding means as illustrated in figure 3, 4 or preferably figure 5. Preferably one overlay comprises two such guiding means each defining a path extending in the surface of the overlay, the paths extending parallel to each other, each on one side of the tooth.

In a fourth and fifth stage, shown in figures 7d and 7e, the edges of the pyramid get rounded. Rounding these edges can be guided by an overlay as shown in figure 1 and 2. Such an overlay comprises a path extending in the surface of the overlay and defining a segment of a circle. Each side of the path is defined by a guiding surface having at least two guiding edges, as illustrated in figure 3. The height of the guiding part 5 of the overlay 1 is defined so as to guide the cutting tool 10 in a predetermined vertical position.

In this succession of stages, the first stage could be executed as the last stage instead of as the first. However tests have shown that starting with smoothening the upper part of the tooth is advantageous in the process of preparing the tooth.

Once the overlay 1 and the guiding means are determined, the determined overlay 1 having the determined guiding means can be produced. This can be done by any known means such as a CAD/CAM system, rapid prototyping or 3D printing. When multiple guiding means have been determined, multiple overlays 1 can be produced each comprising one or several guiding means. Preferably, a rapid prototyping apparatus creates the overlay with a tolerance of about 30 µm.

The overlay 1 has, as already mentioned above, a lower, supporting part 6 and an upper, guiding part 5. As can be seen in the figures, the guiding part 5 has a certain thickness 8, which defines the height 8 of the path and enables the cutting tool to be guided in the vertical way. Preferably the supporting part 6 and/or guiding part 5 comprises a vertical opening 9 from the side to the guiding groove 7, provided to serve as an entrance into the guiding groove 7 for a dental cutting tool. In particular when a cutting tool has a tip portion that has a diameter that is larger than the diameter of the main portion of the cutting tool, for example a high-speed drill with a tip in the form of a ball, this opening is advantageous. Entering such a cutting tool into the guiding groove 7 via the upper part of the overlay would at least partially widen the guiding groove 7 thereby at least partially taking away its possibility to firmly guide the cutting tool along the predetermined path. Furthermore a side entrance 9 is preferred over an entrance via the upper part as it will be easier to enter the cutting tool in a controlled manner into the guiding groove 7.

When a guiding groove 7 is determined to form a circular path in the horizontal plane, it will be preferred to split up this path into several segments, and produce multiple overlays 1 each having a guiding groove 7 corresponding to one segment of this path. This will result in a set of overlays 1 which can be used by a dentist one after the other to remove tooth structure following this circular path. Each overlay 1 of the set of overlays 1 will guide the cutting tool along a particular side of the tooth.

In the embodiment where the predetermined part is not determined based on the shape of the tooth restoration part, the latter can be determined based on the data in the computer. Outer shape of the tooth restoration part can be determined in several ways, all known to the person skilled in the art. A bite lift or other corrective amendment can be directly integrated into the design of the outer shape. A first possibility is to shape the tooth restoration part so that it resembles the outer shape of the original tooth. A second possibility is to shape the tooth restoration part so that it fits with neighbouring teeth. A third possibility is a combination of the first and second, and modifies the shape of the original tooth to better fit with the neighbouring teeth. Inner shape of the tooth restoration part will be determined based on the simulation of the preparation of the tooth. As the tooth will be prepared in a guided manner, it is known in advance what the shape will be of the prepared tooth. The inner shape of the tooth restoration part will be chosen so that it fits the shape of the prepared tooth.

Preferably the method according to the invention further comprises the step of producing a tooth restoration part.

Similar to the determination of the predetermined part, a tooth restoration part can be determined by a computer without any interaction of a dentist. However it will be preferred that the computer determines the tooth restoration part in cooperation with a dentist, for example in a way as described above. The computer can, in this process of determining a tooth restoration part, perform predetermined clinical checks and alert the dentist in case the tooth restoration part does not meet the standard norms. This will enable a dentist to design a tooth restoration part in a fast and reliable manner.

The production of the tooth restoration part can be done by any means known to the skilled person such as a CAD/CAM system.

## Claims

1. Method for producing a dentist tool comprising an overlay (1) to be used by a dentist with a dentist cutting tool in removing tooth structure from a tooth, said method comprising the steps of:
determining a predefined part of tooth structure to be removed so as to prepare said tooth for further treatment;
determining an overlay (1) with a surface;
simulating a movement to be followed by said dentist cutting tool in order to remove a predetermined part of tooth structure from said tooth;
determining a first guiding edge (11) within said overlay (1), said first guiding edge corresponding to said movement;
determining a second guiding edge (12) within said overlay that is spaced apart from said first guiding edge by a distance (d) of at least 0,5mm, said second guiding edge corresponding to said movement, said first and second guiding edges thereby being provided to contact said dentist cutting tool so as to guide said dentist cutting tool such that said dentist cutting tool follows a predetermined tilt and a movement corresponding to said simulated movement for removal of said predetermined part of tooth structure to be removed; and
producing said determined overlay with guiding means which comprises said first and second guiding edges (11,12),
**characterized in that** said surface is configured for fitting said tooth and fitting at least part of a neighbouring tooth of said tooth such that said surface, when so fitted, contacts and surrounds the respective part or parts of said tooth and said neighbouring tooth of said tooth and maintains said overlay in position over said tooth when contacted by said dentist cutting tool due to a normal use of said overlay.

2. Method according to claim 1, wherein the method further comprises the steps of:
processing three-dimensional data relating to a shape of at least said tooth and at least said part of said neighbouring tooth with a computer so as to determine said overlay (1); and
processing said three-dimensional data to determine said predetermined part of tooth structure to be removed.

3. Method according to claim 1 or claim 2, **characterized in that** said first and said second guiding edges are determined for simultaneously contacting said dentist cutting tool at contacting points longitudinally distanced from each other by said distance d, said guiding edges thereby defining a guiding surface configured for guiding said dentist cutting tool.

4. Method according to any of the previous claims, **characterized in that** said guiding means comprise a guiding groove, which defines a path extending in a surface of said overlay and suitable for guiding said dentist cutting tool along said path in such a manner as to enable a guided removal of at least partially said predetermined part of tooth structure.

5. Method for preparing restoration of a tooth structure comprising a method according to any one of the preceding claims, **characterized in that** the method further comprises the steps of:
producing in advance a tooth restoration part having an inner shape determined so as to fit a predetermined remaining part of tooth structure of said tooth after removal of the predetermined part of tooth structure to be removed, and having an outer shape determined so as to fit together with remaining neighbouring teeth.

6. Method for preparing restoration of a tooth structure comprising a method according to claim 2 or according to claim 3 or according to claim 4 where dependent on claim 2, **characterized in that** the method comprises the further steps of:
processing said three-dimensional data to simulate a tooth restoration part having an inner shape determined so as to fit a predetermined remaining part of tooth structure of said tooth after removal of the predetermined part of tooth structure to be removed, and having an outer shape determined so as to fit together with remaining neighbouring teeth; and
producing said tooth restoration part in advance.

7. Method according to claim 6, **characterized in that** said method further comprises:
firstly determining an outer shape of said tooth restoration part so that it fits together with remaining neighbouring teeth, and determining an inner shape of said tooth restoration part that is compatible with said outer shape;
secondly determining a shape of a remaining part of tooth structure of said tooth to remain after removal of the part of tooth structure to be removed so that said inner shape of said tooth restoration part fits said shape of said remaining part of tooth structure of said tooth; and
thirdly determining said predetermined part of tooth structure to be removed as being the part of tooth structure to be removed from said tooth such that said tooth has said shape of said remaining part of tooth structure of said tooth left after removal of the part of tooth structure to be removed.

8. Method according to any one of the preceding claims, **characterized in that** said dental cutting tool comprises a cutting portion and a collar, either or both of said first and second guiding edges being configured to contact said collar along so as to enable said dentist cutting tool to be guided to a predetermined depth.

9. Method according to any one of the preceding claims, **characterized in that** said dentist tool comprises at least one further overlay (1), said overlay and said at least one further overlay being determined to guide a dentist cutting tool in removing tooth structure from a tooth in a plurality of stages, the method further comprising the steps of:
determining at least one further overlay fitting said tooth and fitting at least a part of said neighbouring tooth;
simulating at least one further movement to be followed by said dentist cutting tool in order to remove at least one further predetermined part of said tooth structure to be removed from said tooth;
determining at least one further first guiding edge (11) within said at least one further overlay (1), said at least one further first guiding edge corresponding to said at least one further movement; and
determining at least one further second guiding edge (12) within said at least one further overlay that is spaced apart from said at least one further first guiding edge by a distance (d) of at least 0,5 mm, said at least one further second guiding edge corresponding to said at least one further movement, said at least one further first and second guiding edges thereby being provided to contact said dentist cutting tool so as to guide said dentist cutting tool such that said dentist cutting tool follows at least one further movement corresponding to the simulated movements for removal of said predetermined part of tooth structure to be removed.

10. Method according to claim 9, **characterized in that** said overlay and said at least one further overlay form a part of a set of overlays to be used by a dentist in removing tooth structure for placing a crown or a bridge or an onlay, said set of overlays comprising five overlays in which:
a first overlay is determined for guiding a dentist cutting tool to smoothen an upper part of said tooth based on a desired height of remaining tooth structure after removal;
a second overlay is determined for guiding a dentist cutting tool to cut away one of the longitudinal or transversal sides of the tooth;
a third overlay is determined for guiding a dentist cutting tool to cut away the other of the longitudinal or transversal sides of the tooth;
a fourth overlay is determined for guiding a dentist cutting tool to round off edges between the longitudinal and transversal sides of the tooth at one side of the tooth; and
a fifth overlay is determined for guiding a dentist cutting tool to round off edges between the longitudinal and transversal sides of the tooth at the other side of the tooth.

11. Method according to any of the preceding claims, **characterized in that** said overlay is produced via rapid prototyping.

12. Method according to any one of the preceding claims, **characterized in that** said distance (d) is at least 1 mm.

13. Overlay (1) for contacting a dental cutting tool so as to guide the dental cutting tool in the removal of predetermined tooth structure from a tooth to be treated, said overlay (1) comprising:
an inner surface;
a first guiding edge (11) extending through a thickness of said overlay including through the inner surface, said first guiding edge being configured to contact a dentist cutting tool such that said dentist cutting tool follows a predetermined movement; and
a second guiding edge (12) extending through a thickness of said overlay including through the inner surface, said second guiding edge being spaced apart from said first guiding edge by a distance d of at least 0,5mm and being configured to contact said dentist cutting tool such that said dentist cutting tool follows said predetermined movement and a predetermined tilt, **characterized in that** said inner surface has a shape and dimensions corresponding to an outer surface of the tooth to be treated so that said inner surface is releasably fixable to said tooth and to at least part of a neighbouring tooth of said tooth.

14. Set of overlays to be used for guiding a dental cutting tool in removing predetermined tooth structure from a tooth to be treated, at least one overlay of the set of overlays being the overlay of claim 13, and another one of the set of overlays being:
a first overlay having a shape and dimensions for guiding at least one dental cutting tool to cut away at least a portion of an upper part of a tooth based on a desired height of remaining tooth structure after removal of the upper part of the tooth;
a second overlay having a shape and dimensions for guiding at least one dental cutting tool to cut away at least a portion of a longitudinal side of the tooth;
a third overlay having a shape and dimensions for guiding at least one dental cutting tool to cut away at least a portion of a transversal side of the tooth;
a fourth overlay having a shape and dimensions for guiding at least one dental cutting tool to round off edges between the longitudinal and the transversal sides of the tooth at one side of the tooth; or
a fifth overlay having a shape and dimensions for guiding at least one dental cutting tool to round off edges between the longitudinal and the transversal sides of the tooth at the other side of the tooth opposite the one side of the tooth.

15. A system to be used by a dentist in the removal of predetermined tooth structure from a tooth to be treated, the system comprising:
the overlay of claim 13 or the set of overlays of claim 14; and
a dental cutting tool configured for engaging the overlay of claim 13 or any one of the set of overlays of claim 14.

## Patentansprüche

1. Verfahren zum Herstellen eines Zahnarztwerkzeugs, umfassend eine Auflage (1), die von einem Zahnarzt mit einem Zahnarztschneidwerkzeug beim Entfernen einer Zahnstruktur von einem Zahn zu verwenden ist, wobei das Verfahren die folgenden Schritte umfasst:
Bestimmen eines vorbestimmten Teils der zu entfernenden Zahnstruktur, um den Zahn für die weitere Behandlung vorzubereiten;
Bestimmen einer Auflage (1) mit einer Oberfläche;
Simulieren einer Bewegung, die von dem Zahnarztschneidwerkzeug verfolgt werden soll, um einen vorbestimmten Teil einer Zahnstruktur von dem Zahn zu entfernen;
Bestimmen einer ersten Führungskante (11) innerhalb der Auflage (1), wobei die erste Führungskante der Bewegung entspricht;
Bestimmen einer zweiten Führungskante (12) innerhalb der Auflage, die von der ersten Führungskante um einen Abstand (d) von mindestens 0,5 mm beabstandet ist, wobei die zweite Führungskante der Bewegung entspricht, wobei die erste und zweite Führungskante dadurch bereitgestellt sind, um das Zahnarztschneidwerkzeug zu kontaktieren, um das Zahnarztschneidwerkzeug zu führen, sodass das Zahnarztschneidwerkzeug eine vorbestimmte Neigung verfolgt und eine Bewegung, die der simulierten Bewegung zum Entfernen des vorbestimmten Teils der Zahnstruktur, die entfernt werden soll, entspricht; und
Herstellen der bestimmten Auflage mit einem Führungsmittel, das die erste und zweite Führungskante (11, 12) umfasst,
**dadurch gekennzeichnet, dass** die Oberfläche konfiguriert ist, um den Zahn zu anzupassen und mindestens einen Teil eines benachbarten Zahns des Zahns anzupassen, sodass die Oberfläche, wenn sie derart angepasst ist, den jeweiligen Teil oder die Teile des Zahns und des benachbarten Zahns des Zahns kontaktiert und umgibt, und die Auflage in Position über dem Zahn hält, wenn er von dem Zahnarztschneidwerkzeug infolge einer normalen Verwendung der Auflage kontaktiert wird.

2. Verfahren nach Anspruch 1, wobei das Verfahren weiter die folgenden Schritte umfasst:
Verarbeiten von dreidimensionalen Daten, die sich auf die Form des mindestens einen Zahns und mindestens des Teils des benachbarten Zahns beziehen, mit einem Computer, um die Auflage (1) zu bestimmen; und
Verarbeiten der dreidimensionalen Daten, um den vorbestimmten Teil der Zahnstruktur, die entfernt werden soll, zu bestimmen.

3. Verfahren nach Anspruch 1 oder Anspruch 2, **dadurch gekennzeichnet, dass** die erste und zweite Führungskante bestimmt werden, um das Zahnarztschneidwerkzeug gleichzeitig an Kontaktpunkten zu kontaktieren, die in Längsrichtung im Abstand d voneinander beabstandet sind, wobei die Führungskanten dabei eine Führungsoberfläche definieren, die zum Führen des Zahnarztschneidwerkzeugs konfiguriert sind.

4. Verfahren nach einem der vorstehenden Ansprüche, **dadurch gekennzeichnet, dass** die Führungsmittel eine Führungsnut umfassen, die einen Weg definiert, der sich in eine Oberfläche der Auflage erstreckt und geeignet ist, um das Zahnarztschneidwerkzeug entlang des Weges in einer solchen Weise zu führen, um eine geführte Entfernung von mindestens einem Teil des vorbestimmten Teils der Zahnstruktur zu ermöglichen.

5. Verfahren zum Vorbereiten einer Wiederherstellung einer Zahnstruktur umfassend ein Verfahren nach einem der vorstehenden Ansprüche, **dadurch gekennzeichnet, dass** das Verfahren weiter die folgenden Schritte umfasst:
Herstellen im Vorhinein eines Zahnwiederherstellungsteils, das eine innere Form aufweist, die derart bestimmt ist, um einen vorbestimmten verbleibenden Teil der Zahnstruktur des Zahns nach der Entfernung des vorbestimmten Teils der Zahnstruktur, die entfernt werden soll, anzupassen, und eine äußere Form aufweist, die bestimmt ist, um mit verbleibenden benachbarten Zähnen zusammenzupassen.

6. Verfahren zum Vorbereiten einer Wiederherstellung einer Zahnstruktur umfassend eine Verfahren nach Anspruch 2 oder nach Anspruch 3 oder nach Anspruch 4, wenn er von Anspruch 2 abhängt, **dadurch gekennzeichnet, dass** das Verfahren die weiteren Schritte umfasst:
Verarbeiten der dreidimensionalen Daten, um einen Zahnwiederherstellungsteil zu simulieren, der eine innere Form aufweist, die derart bestimmt ist, um an einen vorbestimmten verbleibenden Teil der Zahnstruktur des Zahns nach der Entfernung des vorbestimmten Teils der Zahnstruktur zu passen, die entfernt werden soll, und der eine äußere Form aufweist, die derart bestimmt ist, um mit den verbleibenden benachbarten Zähnen zusammenzupassen; und
Herstellen des Zahnwiederherstellungsteils im Vorhinein.

7. Verfahren nach Anspruch 6, **dadurch gekennzeichnet, dass** das Verfahren weiter umfasst:
erstens Bestimmen einer äußeren Form des Zahnwiederherstellungsteils, sodass sie mit verbleibenden benachbarten Zähnen zusammenpasst, und Bestimmen einer inneren Form des Zahnwiederherstellungsteils, die mit der äußeren Form kompatibel ist;
zweitens Bestimmen einer Form eines verbleibenden Teils der Zahnstruktur des Zahns, der nach der Entfernung des Teils der Zahnstruktur, die entfernt werden soll, verbleiben soll, sodass die innere Form des Zahnwiederherstellungsteils an die Form des verbleibenden Teils der Zahnstruktur des Zahns passt; und
drittens Bestimmen des vorbestimmten Teils der Zahnstruktur, die entfernt werden soll, als den Teil der Zahnstruktur, die von dem Zahn entfernt werden soll, sodass der Zahn die Form des verbleibenden Teils der Zahnstruktur aufweist, der nach der Entfernung des Teils der Zahnstruktur, die entfernt werden soll, verbleibt.

8. Verfahren nach einem der vorstehenden Ansprüche, **dadurch gekennzeichnet, dass** das Zahnarztschneidwerkzeug einen Schneidabschnitt und einen Bund umfasst, wobei einer oder beide der ersten und zweiten Führungskante konfiguriert sind, um den Bund entlang zu kontaktieren, um zu ermöglichen, dass das Zahnarztschneidwerkzeug auf eine vorbestimmte Tiefe geführt wird.

9. Verfahren nach einem der vorstehenden Ansprüche, **dadurch gekennzeichnet, dass** das Zahnarztwerkzeug mindestens eine weitere Auflage (1) umfasst, wobei die Auflage und die mindestens eine weitere Auflage bestimmt sind, um ein Zahnarztschneidwerkzeug beim Entfernen einer Zahnstruktur von einem Zahn in einer Vielzahl von Stufen zu führen, wobei das Verfahren weiter die Schritte umfasst:
Bestimmen mindestens einer weiteren Auflage, die auf den Zahn passt und auf mindestens einen Teil des benachbarten Zahns passt;
Simulieren mindestens einer weiteren Bewegung, die von dem Zahnarztschneidwerkzeug verfolgt werden soll, um mindestens einen weiteren vorbestimmten Teil der Zahnstruktur zu entfernen, die von dem Zahn entfernt werden soll;
Bestimmen mindestens einer weiteren ersten Führungskante (11) innerhalb der mindestens einen weiteren Auflage (1), wobei die mindestens eine weitere erste Führungskante der mindestens einen weiteren Bewegung entspricht; und
Bestimmen mindestens einer weiteren zweiten Führungskante (12) in der mindestens einen weiteren Auflage, die von der mindestens einen weiteren ersten Führungskante um einen Abstand (d) von mindestens 0,5 mm beabstandet ist, wobei die mindestens eine weitere zweite Führungskante der mindestens einen weiteren Bewegung entspricht, wobei die mindestens eine weitere erste und zweite Führungskante dabei bereitgestellt sind, um das Zahnarztschneidwerkzeug zu kontaktieren, um das Zahnarztschneidwerkzeug zu führen, sodass das Zahnarztschneidwerkzeug mindestens eine weitere Bewegung verfolgt, die den simulierten Bewegungen zum Entfernen des vorbestimmten Teils der Zahnstruktur, die entfernt werden soll, entspricht

10. Verfahren nach Anspruch 9, **dadurch gekennzeichnet, dass** die Auflage und die mindestens eine weitere Auflage einen Teil eines Satzes von Auflagen bilden, der von einem Zahnarzt beim Entfernen von Zahnstruktur verwendet werden soll, um eine Krone oder eine Brücke oder eine Onlay-Plombe zu platzieren, wobei der Satz von Auflagen fünf Auflagen umfasst, von denen:
eine erste Auflage bestimmt ist, um ein Zahnarztschneidwerkzeug zu führen, um einen oberen Teil des Zahns basierend auf einer gewünschten Höhe der verbleibenden Zahnstruktur nach dem Entfernen zu glätten;
eine zweite Auflage bestimmt ist, um ein Zahnarztschneidwerkzeug zu führen, um eine der Längsseite oder Querseite des Zahns wegzuschneiden;
eine dritte Auflage bestimmt ist, um ein Zahnarztschneidwerkzeug zu führen, um die andere der Längsseite oder Querseite des Zahns wegzuschneiden;
eine vierte Auflage bestimmt ist, um ein Zahnarztschneidwerkzeug zu führen, um Kanten zwischen der Längsseite und der Querseite des Zahns an einer Seite des Zahns abzurunden; und
eine fünfte Auflage bestimmt ist, um ein Zahnarztschneidwerkzeug zu führen, um Kanten zwischen der Längsseite und der Querseite des Zahns an der anderen Seite des Zahns abzurunden.

11. Verfahren nach einem der vorstehenden Ansprüche, **dadurch gekennzeichnet, dass** die Auflage mittels schneller Prototypenentwicklung hergestellt wird.

12. Verfahren nach einem der vorstehenden Ansprüche, **dadurch gekennzeichnet, dass** der Abstand (d) mindestens 1 mm ist.

13. Auflage (1) zum Kontaktieren eines Zahnarztschneidwerkzeugs, um das Zahnarztschneidwerkzeug bei der Entfernung einer vorbestimmten Zahnstruktur von einem zu behandelnden Zahn zu führen, wobei die Auflage (1) umfasst:
eine innere Oberfläche;
eine erste Führungskante (11), die sich durch eine Dicke der Auflage, einschließlich durch die innere Oberfläche erstreckt, wobei die erste Führungskante konfiguriert ist, um ein Zahnarztschneidwerkzeug derart zu kontaktieren, dass das Zahnarztschneidwerkzeug eine vorbestimmte Bewegung verfolgt; und
eine zweite Führungskante (12), die sich durch eine Dicke der Auflage, einschließlich durch die innere Oberfläche erstreckt, wobei die zweite Führungskante von der ersten Führungskante in einem Abstand d von mindestens 0,5mm beabstandet ist und konfiguriert ist, um das Zahnarztschneidwerkzeug derart zu kontaktieren, dass das Zahnarztschneidwerkzeug die vorbestimmte Bewegung und eine vorbestimmte Neigung verfolgt,
**dadurch gekennzeichnet, dass** die innere Oberfläche eine Form und Dimensionen aufweist, die einer äußeren Oberfläche des Zahns entsprechen, der behandelt werden soll, sodass die innere Oberfläche entfernbar an den Zahn und an mindestens einen Teil des benachbarten Zahns des Zahns befestigt werden kann.

14. Satz von Auflagen, die zum Führen mindestens eines Zahnarztschneidwerkzeugs beim Entfernen einer vorbestimmten Zahnstruktur von einem Zahn, der behandelt werden soll zu verwenden sind, wobei mindestens eine Auflage des Satzes von Auflagen die Auflage von Anspruch 13 ist, und eine andere des Satzes von Auflagen Folgende sind:
eine erste Auflage, die eine Form und Dimensionen aufweist, um mindestens ein Zahnarztschneidwerkzeug zu führen, um mindestens einen Abschnitt eines oberen Teils eines Zahns basierend auf einer gewünschten Höhe der verbleibenden Zahnstruktur nach dem Entfernen des oberen Teils des Zahns wegzuschneiden;
eine zweite Auflage, die eine Form und Dimensionen aufweist, um mindestens ein Zahnarztschneidwerkzeug zu führen, um mindestens einen Abschnitt einer Längsseite des Zahns wegzuschneiden;
eine dritte Auflage, die eine Form und Dimensionen aufweist, um mindestens ein Zahnarztschneidwerkzeug zu führen, um mindestens einen Abschnitt einer Querseite des Zahns wegzuschneiden;
eine vierte Auflage, die eine Form und Dimensionen aufweist, um mindestens ein Zahnarztschneidwerkzeug zu führen, um Kanten zwischen der Längsseite und der Querseite des Zahns an einer Seite des Zahns abzurunden; oder
eine fünfte Auflage, die eine Form und Dimensionen aufweist, um mindestens ein Zahnarztschneidwerkzeug zu führen, um Kanten zwischen der Längsseite und der Querseite des Zahns an der anderen Seite des Zahns abzurunden, der gegenüber der einen Seite des Zahns liegt.

15. System, das von einem Zahnarzt bei der Entfernung einer vorbestimmten Zahnstruktur von einem zu behandelnden Zahn zu verwenden ist, wobei das System umfasst:
die Auflage nach Anspruch 13 oder den Satz von Auflagen nach Anspruch 14; und
ein Zahnarztschneidwerkzeug, das konfiguriert ist, um in die Auflage nach Anspruch 13 oder in eine des Satzes von Auflagen nach Anspruch 14 einzugreifen.

## Revendications

1. Procédé de production d'un outil de dentiste comprenant un recouvrement (1) à utiliser par un dentiste avec un outil de coupe de dentiste pour la dépose d'une structure dentaire d'une dent, ledit procédé comprenant les étapes consistant à :
déterminer une partie prédéfinie de structure dentaire à enlever de façon à préparer ladite dent pour un traitement ultérieur ;
déterminer un recouvrement (1) avec une surface ;
simuler un mouvement à suivre par ledit outil de coupe de dentiste afin d'enlever une partie prédéterminée de structure dentaire de ladite dent ;
déterminer un premier bord de guidage (11) dans ledit recouvrement (1), ledit premier bord de guidage correspondant audit mouvement ;
déterminer un second bord de guidage (12) dans ledit recouvrement qui est espacé dudit premier bord de guidage d'une distance (d) d'au moins 0,5 mm, ledit second bord de guidage correspondant audit mouvement, lesdits premier et second bords de guidage étant ainsi prévus pour entrer en contact avec ledit outil de coupe de dentiste de façon à guider ledit outil de coupe de dentiste de sorte que ledit outil de coupe de dentiste suive une inclinaison prédéterminée et un mouvement correspondant audit mouvement simulé pour la dépose de ladite partie prédéterminée de structure dentaire à enlever ; et
produire ledit recouvrement déterminé avec des moyens de guidage qui comprennent lesdits premier et second bords de guidage (11, 12),
**caractérisé en ce que** ladite surface est configurée pour s'adapter à ladite dent et s'adapter au moins en partie à une dent voisine de ladite dent, de sorte que ladite surface, lorsqu'elle est ainsi installée, entre en contact et entoure la ou les parties respectives de ladite dent et ladite dent voisine de ladite dent et maintienne ledit recouvrement en position sur ladite dent lorsqu'elle est mise en contact avec ledit outil de coupe de dentiste du fait d'une utilisation normale dudit recouvrement.

2. Procédé selon la revendication 1, dans lequel le procédé comprend en outre les étapes consistant à :
traiter des données en trois dimensions relatives à une forme d'au moins ladite dent et au moins ladite partie de ladite dent voisine avec un ordinateur afin de déterminer ledit recouvrement (1) ; et
traiter lesdites données en trois dimensions pour déterminer ladite partie prédéterminée de structure dentaire à enlever.

3. Procédé selon la revendication 1 ou la revendication 2, **caractérisé en ce que** ledit premier et ledit second bords de guidage sont déterminés pour entrer en contact simultanément avec ledit outil de coupe de dentiste au niveau des points de contact espacés longitudinalement les uns des autres de ladite distance d, lesdits bords de guidage définissant ainsi une surface de guidage configurée pour guider ledit outil de coupe de dentiste.

4. Procédé selon l'une quelconque des revendications précédentes, **caractérisé en ce que** lesdits moyens de guidage comprennent une cannelure de guidage, qui définit un chemin s'étendant dans une surface dudit recouvrement et apte à guider ledit outil de coupe de dentiste le long dudit chemin, de façon à permettre une dépose guidée d'au moins partiellement ladite partie prédéterminée de la structure dentaire.

5. Procédé de préparation d'un rétablissement d'une structure dentaire comprenant un procédé selon l'une quelconque des revendications précédentes, **caractérisé en ce que** le procédé comprend en outre les étapes consistant à :
produire en avance une partie de rétablissement de dent ayant une forme intérieure déterminée de façon à s'adapter à une partie restante prédéterminée de la structure dentaire de ladite dent après dépose de la partie prédéterminée de la structure dentaire à enlever, et ayant une forme extérieure déterminée de façon à s'adapter ensemble aux dents voisines restantes.

6. Procédé de préparation du rétablissement d'une structure dentaire comprenant un procédé selon la revendication 2 ou selon la revendication 3 ou selon la revendication 4, dépendantes de la revendication 2, **caractérisé en ce que** le procédé comprend les étapes ultérieures consistant à :
traiter lesdites données en trois dimensions afin de simuler une partie de rétablissement d'une dent ayant une forme intérieure déterminée, de façon à s'adapter à une partie restante prédéterminée d'une structure de dent de ladite dent après dépose de la partie prédéterminée de structure dentaire à enlever, et ayant une forme extérieure déterminée de façon à s'adapter ensemble avec les dents voisines restantes ; et
produire ladite partie de rétablissement de dent en avance.

7. Procédé selon la revendication 6, **caractérisé en ce que** ledit procédé comprend en outre :
la détermination en premier lieu d'une forme extérieure de ladite partie de rétablissement de dent de sorte qu'elle s'adapte ensemble aux dents voisines restantes, et la détermination d'une forme intérieure de ladite partie de rétablissement de dent qui soit compatible avec ladite forme extérieure ;
la détermination en second lieu d'une forme d'une partie restante de structure dentaire de ladite dent devant rester après la dépose de la partie de structure dentaire à enlever, de sorte que ladite forme intérieure de ladite partie de rétablissement de dent s'adapte à ladite forme de ladite partie restante de structure dentaire de ladite dent ; et
la détermination en troisième lieu de ladite partie prédéterminée de structure dentaire à enlever comme faisant partie d'une structure dentaire à enlever de ladite dent de sorte que ladite dent ait ladite forme de ladite partie restante de structure dentaire de ladite dent laissée après dépose de la partie de structure dentaire à enlever.

8. Procédé selon l'une quelconque des revendications précédentes, **caractérisé en ce que** ledit outil de coupe dentaire comprend une partie de coupe et un collier, l'un ou les deux desdits premier et second bords de guidage étant configurés pour entrer en contact avec ledit collier de façon à permettre audit outil de coupe de dentiste d'être guidé jusqu'à une profondeur prédéterminée.

9. Procédé selon l'une quelconque des revendications précédentes, **caractérisé en ce que** ledit outil de dentiste comprend au moins un recouvrement ultérieur (1), ledit recouvrement et ledit au moins un recouvrement ultérieur étant déterminés pour guider un outil de coupe de dentiste dans la dépose d'une structure dentaire d'une dent dans une pluralité de phases, le procédé comprenant en outre les étapes consistant à :
déterminer au moins un recouvrement ultérieur s'adaptant à ladite dent et s'adaptant au moins partiellement à ladite dent voisine ;
simuler au moins un mouvement ultérieur à suivre par ledit outil de coupe de dentiste afin d'enlever au moins une autre partie prédéterminée de ladite structure dentaire à enlever de ladite dent ;
déterminer au moins un autre premier bord de guidage (11) dans ledit au moins un recouvrement ultérieur (1), ledit au moins un premier bord de guidage ultérieur correspondant audit au moins un mouvement ultérieur ; et
déterminer au moins un second bord de guidage ultérieur (12) dans ledit au moins un recouvrement ultérieur qui est espacé dudit au moins un premier bord de guidage ultérieur d'une distance (d) d'au moins 0,5 mm, ledit au moins un second bord de guidage ultérieur correspondant audit au moins un mouvement ultérieur, lesdits au moins un premier et un second bords de guidage ultérieurs étant ainsi prévus pour entrer en contact avec ledit outil de coupe de dentiste, de façon à guider ledit outil de coupe de dentiste de sorte que ledit outil de coupe de dentiste suive au moins un mouvement ultérieur correspondant aux mouvements simulés pour dépose de ladite partie prédéterminée de structure dentaire à enlever.

10. Procédé selon la revendication 9, **caractérisé en ce que** ledit recouvrement et ledit au moins un recouvrement ultérieur forment une partie d'un ensemble de recouvrements à utiliser par un dentiste dans la dépose d'une structure dentaire pour placer une couronne ou un bridge ou un onlay, ledit ensemble de recouvrements comprenant cinq recouvrements dans lesquels :
un premier recouvrement est déterminé pour guider un outil de coupe d'un dentiste pour lisser une partie supérieure de ladite dent sur la base d'une hauteur souhaitée de structure dentaire restante après dépose ;
un deuxième recouvrement est déterminé pour guider un outil de coupe de dentiste afin de couper un parmi le côté longitudinal ou transversal de la dent ;
un troisième recouvrement est déterminé pour guider un outil de coupe de dentiste afin de couper l'autre parmi le côté longitudinal ou transversal de la dent ;
un quatrième recouvrement est déterminé pour guider un outil de coupe de dentiste pour arrondir les bords entre les côtés longitudinal et transversal de la dent d'un côté de la dent ; et
un cinquième recouvrement est déterminé pour guider un outil de coupe de dentiste pour arrondir des bords entre les côtés longitudinal et transversal de la dent de l'autre côté de la dent.

11. Procédé selon l'une quelconque des revendications précédentes, **caractérisé en ce que** ledit recouvrement est produit par le biais d'un prototypage rapide.

12. Procédé selon l'une quelconque des revendications précédentes, **caractérisé en ce que** ladite distance (d) est d'au moins 1 mm.

13. Recouvrement (1) pour entrer en contact avec un outil de coupe dentaire de façon à guider l'outil de coupe dentaire dans la dépose de structure dentaire prédéterminée d'une dent à traiter, ledit recouvrement (1) comprenant :
une surface interne ;
un premier bord de guidage (11) s'étendant à travers une épaisseur dudit recouvrement y compris à travers la surface interne, ledit premier bord de guidage étant configuré pour entrer en contact avec un outil de coupe de dentiste de sorte que ledit outil de coupe de dentiste suive un mouvement prédéterminé ; et
un second bord de guidage (12) s'étendant à travers une épaisseur dudit recouvrement y compris à travers la surface interne, ledit second bord de guidage étant espacé dudit premier bord de guidage d'une distance d d'au moins 0,5 mm et étant configuré pour entrer en contact avec ledit outil de coupe de dentiste de sorte que ledit outil de coupe de dentiste suive ledit mouvement prédéterminé et une inclinaison prédéterminée,
**caractérisé en ce que** ladite surface interne a une forme et des dimensions correspondant à une surface extérieure de la dent à traiter de sorte que ladite surface interne peut être fixée de manière amovible à ladite dent et à au moins une partie d'une dent voisine de ladite dent.

14. Ensemble de recouvrements à utiliser pour guider un outil de coupe dentaire dans la dépose d'une structure dentaire prédéterminée d'une dent à traiter, au moins un recouvrement de l'ensemble de recouvrements étant le recouvrement selon la revendication 13, et un autre parmi l'ensemble de recouvrements étant :
un premier recouvrement ayant une forme et des dimensions pour guider au moins un outil de coupe dentaire pour couper au moins une portion d'une partie supérieure d'une dent sur la base d'une hauteur souhaitée de la structure de dent restante après dépose de la partie supérieure de la dent ;
un second recouvrement ayant une forme et des dimensions pour guider au moins un outil de coupe dentaire pour enlever au moins une partie d'un côté longitudinal de la dent ;
un troisième recouvrement ayant une forme et des dimensions pour guider au moins un outil de coupe dentaire pour enlever au moins une partie d'un côté transversal de la dent ;
un quatrième recouvrement ayant une forme et des dimensions pour guider au moins un outil de coupe dentaire pour arrondir des bords entre les côtés longitudinal et transversal de la dent d'un côté de la dent ; ou
un cinquième recouvrement ayant une forme et des dimensions pour guider au moins un outil de coupe dentaire pour arrondir des bords entre les côtés longitudinal et transversal de la dent de l'autre côté de la dent, à l'opposé d'un côté de la dent.

15. Système à utiliser par un dentiste dans la dépose d'une structure dentaire prédéterminée d'une dent à traiter, le système comprenant :
le recouvrement selon la revendication 13, ou l'ensemble de recouvrements selon la revendication 14 ; et
un outil de coupe dentaire configuré pour mettre en prise le recouvrement selon la revendication 13, ou l'un quelconque de l'ensemble de recouvrements selon la revendication 14.
